# EUROPEAN PATENT SPECIFICATION

(11) **EP 0 573 141 B1**
(45) Date of publication and mention of the grant of the patent: **06.10.1999**
(21) Application number: 93302609.8
(22) Date of filing: 01.04.1993
(51) Int. Cl.: A61K 7/06

(54) **External use of a hair restorer containing pine extract**
Äusserliche Anwendung eines Kiefernextrakt enthaltenden, haarregenerierenden Mittels
Usage externe d'un agent de régénération des cheveux contenant un extrait de pin

(30) Priority: 04.06.1992 JP 18562092
(43) Date of publication of application: 08.12.1993
(73) Proprietor: KABUSHIKI KAISHA HAYASHIBARA SEIBUTSU KAGAKU KENKYUJO, Okayama-shi Okayama (JP)
(72) Inventor: Uchida, Yukio, Okayama-shi, Okayama (JP); Iritani, Satoshi, Akaiwa-gun, Okayama (JP); Miyake, Toshio, Okayama-shi, Okayama (JP)
(74) Representative: Daniels, Jeffrey Nicholas

(56) References cited:
- BE-A- 665 257
- FR-A- 2 318 620
- PATENT ABSTRACTS OF JAPAN vol. 1, no. 59 (C-015) & JP-A-52 018 833 (YOSHIO SASAKI)
- PATENT ABSTRACTS OF JAPAN vol. 6, no. 257 (C-140) & JP-A-57 150 620 (AKIRA IZUNO)
- VOIGT: "Lehrbuch der pharmazeutischen technologie, 5. aufl." 1, 1984, VEB VERLAG VOLK UND GESUNDHEIT, BERLIN

## Description

The present invention relates to an externally-usable hair restorer containing a pine extract and having an augmented effect of the growth and regeneration of hair.

The present inventors has established an orally administrable hair restorer containing the extract of a pine, bamboo and Japanese apricot as disclosed in Japanese Patent Application No.126,605/92.

As described in the thirty-fourth volume of "Pine", the section of tree, "Honzokoumoku", a Chinese medicinal herb guidebook, it is well known that a pine extract has been used for a long time as a hair restorer. Recently, for instance, a hair restorer utilizing a pine extract is disclosed in Japanese Patent Laid-Open No.164,811/90.

However, the present inventors studied and found that a satisfiable effect of the growth and regeneration of hair is unattainable by using only a pine extract and an external-usable hair restorer is hard to be established by the same. It has been a great demand to establish a highly-safe and external-usable hair restorer containing a pine extract as well as exhibiting an excellent effect of the growth and regeneration of hair.

It would be one of our common wishes to keep our head with capilli during our life.

The present invention has an object to establish a highly-safe and external-usable hair restorer containing a pine extract and exhibiting an excellent effect of the growth and regeneration of hair for applicable to human and animals.

The present invention was made to overcome the above object. The present inventors studied to establish an external-usable hair restorer containing a pine extract and having an augmented effect of said pine extract by incorporating in it various plant extracts which have been used as a folk medicine.

As a result, we unexpectedly found that the present object is attainable by incorporating to a pine extract either or both of a bamboo extract and a Japanese apricot extract which exhibits hardly per se an effect of the growth and regeneration of hair, and accomplished the present invention.

Accordingly, the present invention provides use of a composition containing as effective ingredients a pine extract and either or both of a bamboo extract and a Japanese apricot extract for the manufacture of a medicament for the restoration of hair loss by the external application of the medicament.

The present invention also provides a method of restoring hair of a human which comprises externally applying to said human a composition which contains as effective ingredients a pine extract and either or both of a bamboo extract and a Japanese apricot extract so that a cosmetic restoration of hair occurs.

We found that an external-usable hair restorer containing a pine extract in & concentration of at least 0.01 w/w % (the wording "w/w %" as referred to in the invention will be abbreviated as "%" hereinafter, unless specified otherwise) and a bamboo extract and a Japanese apricot extract in the range of 0.1 to 100 fold that of the pine extract, respectively on a dry solid basis (d.s.b.), is desirable, and accomplished the present invention.

Furthermore, we found that the effect of the growth and regeneration of hair of said external-usable hair restorer is augmented by incorporating one or more a member selected from the group consisting of α-glycosyl-L-ascorbic acid, α-glycosyl-bioflavonoid, propolis extract and cyanine dyes, in addition to a pine extract and either or both of a bamboo extract and a Japanese apricot extract, and accomplished the present invention.

The present invention will be described in further detail hereinafter with reference to the Examples.

The pine extracts usable in the invention include those which are prepared by extracting leaves, branches, seeds, cones and barks of pines, as well as their tissue cultures. The extraction is not restricted, and conventional technique can be suitable employed.

For example, a method using water or a readily water-soluble organic solvent as disclosed in Japanese Patent Laid-Open No.21,060/88, and a method using an aqueous alkaline solution as disclosed in Japanese Patent Laid-Open No.238,532/89 are usually employed.

If necessary, a method as disclosed in Japanese Patent Laid-Open No.172,096/88, wherein a liquefied carbon dioxide in a supercritical state is used, can be employed.

The pine extracts thus obtained are in the form of liquid, paste or powder, and these forms can be adequately chosen to meet a final application. Commercially available pine extracts can be advantageously used, if necessary.

The bamboo extracts usable in the invention include those which are prepared by extracting leaves, branches and roots of bamboos including grass bamboos and the extraction is not restricted and conventional techniques can be used similarly as in the case of pine extracts. If necessary, commercially available sasa extracts or extracts of bamboo grasses such as concentrated extracts of "Kumazasa" (plants of the species Sasa albo-marginata) extracted with hot water can be advantageously used.

The Japanese apricot extracts usable in the invention include those which are prepared by extracting a flesh of Japanese apricot. The extraction is not restricted and conventional techniques can be advantageously used. If necessary, commercially available Japanese apricot extracts such as food products thereof can be advantageously used.

The α-glycosyl-L-ascorbic acid, α-glycosyl-bioflavonoid and cyanine dyes usable in the invention mean a compound as disclosed in Japanese Patent Laid-Open No.46,112/92, specifically, said α-glycosyl-L-ascorbic acid has a structure wherein one or more α-D-glucosyl residues are bound in α-1,4 fashion to the hydroxy group of alcohol at the C-2 position in L-ascorbic acid, for instance, those as disclosed in Japanese Laid-Open Nos.135,992/91 and 139,288/91.

The α-glycosyl-L-ascorbic acid, may be 2-0-α-glucosyl-L-ascorbic acid, 2-0-α-maltosyl-L-ascorbic acid, 2-0-α-maltotriosyl-L-ascorbic acid, 2-0-α-maltotetraosyl-L-ascorbic acid, 2-0-α-maltopentaosyl-L-ascorbic acid, 2-0-α-meltohexaosyl-L-ascorbic acid, 2-0-α-maltoheptaosyl-L-ascorbic acid or a mixture thereof.

The α-glycosyl bioflavonoid usable in the invention may be an α-glycosyl rutin, an α-glycosyl hesperidin an α-glycosyl naringin or mixtures thereof and has a structure wherein equimolar or more D-glucose residues are bound in α-fashion to bioflavonoid such as rutin, hesperidin and naringin, for example, those as disclosed in Japanese Patent Laid-Open Nos. 27,293/91, 115,292/91, 7,593/91 and 13,691/92. The cyanine dyes favourably usable in the invention are, for example, the photosensitizing dye "Kankoh-Shikiso No.301 and Kankoh-Shikiso No.401" commercialized by Nippon Kankoh-Shikiso Kenkyusho Co., Okayama, Japan.

The α-glycosyl rutin usable in the present invention is preferably α-glucosyl rutin, α-maltosyl rutin, α-maltotriosyl rutin, α-maltotetraosyl rutin, α-maltopentaosyl rutin, or a mixture thereof.

The α-glycosyl hesperidin usable in the present invention is preferably α-glucosyl hesperidin, α-maltosyl hesperidin, α-maltotriosyl hesperidin, α-maltotetraosyl hesperidin, α-maltopentaosyl hesperidin, or mixtures thereof.

The α-glycosyl naringin usable in the present invention is preferably α-glucosyl naringin, α-maltosyl naringin, α-maltotriosyl naringin, α-maltotetraosyl naringin or mixtures thereof.

The propolis extracts usable in the invention include those in the liquid form which are prepared by extracting propolis into a readily water-soluble organic solvent such as ethanol in the form of liquid and in the powder form thereof, for example, propolis extracts as disclosed in Japanese Patent Application No.166,538/91 and 296,698/91 can be advantageously used.

The external-usable hair restorer containing a pine extract used in the invention is an external-usable hair restorer containing either or both of a bamboo extract and a Japanese apricot extract together with a pine extract and exhibiting an effect of the growth and regeneration of hair.

The suitable amount of a pine extract is at least 0.01%, preferably, in the range of 0.02 to 5%. In case that the amount of a pine extract is less than 0.01%, the inherent effect of the pane extract would be expected, while said effect is deteriorated by a bamboo extract and a Japanese apricot extract when the amount of the pine extract exceeds 5%, d.s.b. Neither a bamboo extract nor a Japanese apricot extract exhibits per se an effect of the growth and regeneration of hair, however said effect is extremely exhibited and augmented in combination with a pine extract. The amounts of a bamboo extract and a Japanese apricot extract against that of a pine extract suitably used in the invention are respectively about 0.1 to 100 fold, d.s.b., preferably, in the range of about 0.1 to 20 fold, d.s.b. for the augmentation of the effect of the growth and regeneration of hair.

The amounts of α-glycosyl ascorbic acid, α-glycosyl bioflavonoid and propolis extract against that of a pine extract suitably used in the invention are respectively in the range of about 0.1 to 100.0 fold, d.s.b.

The amounts of cyanine dyes against that of a pine extract suitably used in the invention is in the range of about 0.01 to 1.0 fold, d.s.b.

The external-usable hair restorer of the invention is preferably adjusted to an aqueous solution containing the above essential ingredients totally in a concentration of about 0.1 to 30%, desirably in the range of about 0.5 to 20%. The hair restorer can be advantageously adjusted into an adequate concentration depending on sort of the ingredients to be incorporated and of hair diseases to be treated, if necessary, and the hair restorer can be prepared into an adequate form, for example, liquid, jelly, emulsion, aerosol or paste.

In addition to the above essential ingredients, for example, an oil- or water-base, emollient, emulsifier, gelatinizer, flavoring agent, antiseptic, antioxidant, coloring agent, refrigerant, bactericide, humectant and pH adjuster, which have been used in conventional hair restorers, can be suitably incorporated in the present hair restorer, if necessary.

The humectant used in the present invention is preferably propylene glycol, glycerine or polyoxyethylene glycol.

Furthermore, the present hair restorer can be used in combination with other ingredients, for example, vitamins, hormones, amino acids, vasodilator, blood-circulation promoting agents, cell activators, antiinflammatories, hyperergic agents for skin, and keratolytic, which have an effect of the growth and regeneration of hair.

The external-usable hair restorer according to the present invention is used as an adequate hair-care product, for example, hair restorer, hair tonic, hair liquid, hair lotion, hair cream, hair oil, hair treatment, hair mousse, shampoo, rinse and oniment. The hair restorer of the invention is commercially valuable because it stables over a relatively long period of time, and is excellent in the promotion of the growth and regeneration of hair when applied to human skin.

Thus, the present external-usable hair restorer accelerates the growth and regeneration of hair and betters the symptom of alopecias, for example, juvenile alopecia, premature alopecia, senile alopecia, alopecia areata, mechanical alopecia, and symptomatic alopecia, as well as exhibiting a satisfiable preventive and therapeutic effect on dandruff, poliosis, and itching of the scalp or skin.

Furthermore, the present hair restorer imparts a commercial value to fur-animals such as sheep, fox, alpaca, angora rabbit, mink and cashmere goat whose hair and fur are utilized. The hair restorer can be utilized for improving the quality of hair or plumage of pet animals such as dog, cat, parrakeet and canary, and enriching the hair gloss of such animals.

The present external-usable hair restorer is usually administered 1 to 3 times a day to hair sites.

If necessary, the effect of the growth and regeneration of hair can be more augmented by iontophoresing an ionized ingredients into affected sites with the low-frequency therapeutic apparatuses as disclosed in Japanese Patent Publication No.41,747/84 and Japanese Patent Laid-Open No.56,060/89.

The following experiments will explain the present invention in detail.

### Experiment 1

### Influence of plant extracts on effect of the growth and regeneration of hair of pine extract

The effect of the growth and regeneration of hair of pine extracts and plant extracts as well as the mixture thereof was evaluated.

The pine extract was in usual manner prepared from a pine leaf and other plant extracts were prepared from commercially available ones or prepared in usual manner, respectively dissolved to give a concentration of 1.0% in a 50.0% aqueous ethanol solution containing 2.0% propyleneglycol. The resultant was adjusted to pH 6.7 by the addition of hydrochloric acid or sodium hydroxide to obtain a solution in hair lotion form. As a control, a solution in hair lotion form was prepared similarly as above, except for the plant extracts were replaced with refined water.

The dorsum surface of rabbits was sectioned into four areas, 5cm² each, i.e. two area near at head side and two areas near at tail side which were respectively bisymmetrical about the vertebral columns of the rabbits, the four areas were depilated by applying thereto silver cream and subjecting them to brief standing. The depilated areas were first sufficiently washed with water, then the depilated areas of the right area near at head side, the left area near at head side, and the right area near at tail side were respectively applied once every day by using a paintbrush with 1ml of the test solution containing only an extract, 1ml of the control solution containing refined water, and 1ml of the test solution containing the mixture of extracts. Furthermore, the left area near at tail side was untreated.

The application of each solution was started on the second day after the depilation, and the newly regenerated hairs were depilated to count on the 10th and 20th days after the application. The length of 10 coarse hairs regenerated in each area was measured with a micromanipulator, and expressed by the mean remainder of hair length (mm) between the hair length in the area which had been applied with a sample solution and that in the area which had been untreated.

In addition, it has been known that the growth rates of hairs in the bisymmetrical dorsum surface about the vertebral column of rabbit is in the same level, and those in both sexes are also in the same level.

The results were as shown in Table 1.

As evidence from the results in Table 1, a synergistical improvement of the effect of the growth and regeneration of hair was found in the groups of the areas

**Table 1**

| Test No. | | Mean remainder of hair length (mm) | |
|---|---|---|---|
| | | 10th | 20th |
| 1. | Pine extract | 0.3 | 0.5 |
| 2. | Pine extract + loquat (Eriobotrya japonica) leaf extract | 0.1 | 0.1 |
| 3. | Dokudami (Houttuynia cordata) extract | 0.1 | 0.1 |
| 4. | Chlorella extract | 0.1 | 0.1 |
| 5. | Bamboo extract | 0.1 | 0.2 |
| 6. | Suzuna (Arabis flagellosa Miq.) extract | 0.1 | 0.1 |
| 7. | Japanese apricot extract | 0.1 | 0.2 |
| 8. | Pine extract + loquat leaf extract | 0.2 | 0.5 |
| 9. | Pine extract + dokudami extract | 0.3 | 0.6 |
| 10. | Pine extract + chlorella extract | 0.3 | 0.5 |
| 11. | Pine extract + bamboo extract | 0.5 | 1.0 |
| 12. | Pine extract + suzuna extract | 0.3 | 0.5 |
| 13. | Pine extract + Japanese apricot extract | 0.6 | 1.1 |
| 14. | Refined water | 0.0 | 0.0 |

which had been applied with the sample solution containing pine extracts in combination with bamboo extracts and Japanese apricot extracts.

### Experiment 2

### Influence of bamboo extract and Japanese apricot extract on an effect of the growth and regeneration of hair of pine extract

By using the sample solutions containing bamboo extracts and Japanese apricot extracts together with pine extracts, the effect of the growth and regeneration of hair was evaluated by the same method as in Experiment 1.

Samples used in the panel test were prepared by the same method as that in Experiment 1 by first providing a group consisting of 0.02%, 0.2% and 2.0% of a pine extract, then adding the groups 0.1 to 100 fold volumes of a bamboo extract and a Japanese apricot extract against the weight of the pine extract, respectively d.s.b.

The application term of each solution was extended to 30 days after the depilation, and the newly regenerated hairs were measured on the 20th and 30th days after the application at the same way of Experiment 1, and expressed by the mean remainder of hair length (mm) in between the applied and the untreated area.

The results were as shown in Table 2.

As evidence from the results in Table 2, an extremely improvement of the effect of the growth and regeneration of hair of a pine extract was found in the groups of the areas which had been applied with the sample solution containing pine extracts in combination with bamboo extracts and

**Table 2**

| Test No. | | Magnification against pine extract, d.s.b. | | Mean remainder of hair lenght (mm) | |
|---|---|---|---|---|---|
| | | | | 20th | 30th |
| 1. | Pine extract 0.02% + | Bamboo extract | 0 | | |
| | | Japanese apricot extract | 0 | 0.1 | 0.2 |
| 2. | Pine extract 0.02% + | Bamboo extract | 10.0 | | |
| | | Japanese apricot extract | 10.0 | 0.3 | 0.5 |
| 3. | Pine extract 0.02% + | Bamboo extract | 100.0 | | |
| | | Japanese apricot extract | 100.0 | 0.6 | 0.7 |
| 4. | Pine extract 0.2% + | Bamboo extract | 0 | | |
| | | Japanese apricot extract | 0 | 0.3 | 0.4 |
| 5. | Pine extract 0.2% + | Bamboo extract | 1.0 | | |
| | | Japanese apricot extract | 1.0 | 1.1 | 1.7 |
| 6. | Pine extract 0.2% + | Bamboo extract | 10.0 | | |
| | | Japanese apricot extract | 10.0 | 1.5 | 1.9 |
| 7. | Pine extract 2.0% + | Bamboo extract | 0 | | |
| | | Japanese apricot extract | 0 | 0.7 | 0.8 |
| 8. | Pine extract 2.0% + | Bamboo extract | 0.1 | | |
| | | Japanese apricot extract | 0.1 | 1.5 | 2.0 |
| 9. | Pine extract 2.0% + | Bamboo extract | 1.0 | 1.8 | 2.2 |
| | | Japanese apricot extract | 1.0 | | |
| 10. | Refined water | | | 0.0 | 0.0 |

Japanese apricot extracts.

### Experiment 3

### Clinical test

By using the No.6 and No.8 specimens which had been exhibited an satisfiable effect of the growth and regeneration of hair in Experiment 2, volunteers with alopecia as the recipient received 3-month clinical test. As a control, the No.10 specimen prepared by the method in Experiment 2 was used. The volunteers were 20 randomly chosen patients with alopecias (10 men and 10 women) and 18 to 62 years old.

The symptom of the volunteers, who had received a pharmacotherapy or physiotherapy at a department of dermatology, were the male alopacia such juvenile alopecia, premature alopecia, senile alopecia, and alopecia areata such as multiple alopecia areata and malignant alopecia areata.

Since the symptom of the volunteers with a slight alopecia areata may be spontaneously recovered, the volunteers with a relatively small alopecic site and those with the atrophia cutis or atrophic pores which were not clinically observed were excluded in this Experiment.

The procedure of therapeutic treatment used in this Experiment is carried out by applying to the affected sites of the volunteers a thin coat of the hair restorer, massaging the sites sufficiently and exposed to an artificial sunlight. Furthermore the volunteers were instructed to apply the hair restorer to the affected sites 3 times a day at home.

The efficacy of the growth and regeneration of hair attained by the hair restorer was evaluated based on the four ranks, i.e. "recovery (hair was newly regenerated to exhibit apparently free from alopecia)", "moderate recovery (though the rate of hair regeneration was moderate, no recurrence of alopecia was found)", "not effected (no regeneration of hair was found)", and "unfavorable (side effect or the acceleration of alopecia was found)".

The results were as shown in Table 3.

As evident from the results in Table 3, similar to the animal test in Experiment 2, the sample solution containing pine extracts together with bamboo extracts and Japanese apricot extracts exhibited an extremely improvement of the effect of the growth and regeneration of hair, and exhibited a satisfiable therapeutic effect against various alopecias. Furthermore, no side effect of the sample solution was found.

### Experiment 4

### Acute toxicity test

Samples No.11 and No.13 prepared by the method in Experiment 1 and samples No.3, No.6 and No.9 prepared by the method in Experiment 2 were tested for the acute toxicity by administering respectively the samples to 7-week-old dd-strain mice.

No mouse died up to the dose of 5g/rat, and a higher dose test was impossible. The results revealed that the acute toxicities of the samples were extremely low.

The preferred examples of the present invention will be described hereinafter.

**Table 3**

| Effective ingredients | | Therapeutic efficacy (Mumber of volunteers) | | | | Judgement |
|---|---|---|---|---|---|---|
| | | Recovery | Moderate recovery | Note effected | Unfavorable | |
| Pine extract | 0.2% | 4 | 12 | 4 | 0 | Present Invention |
| Bamboo extract | 2.0% | | | | | |
| Japanese apricot extract | 2.0% | | | | | |
| Pine extract | 2.0% | 6 | 11 | 3 | 0 | Present Invention |
| Bamboo extract | 0.2% | | | | | |
| Japanese apricot extract | 0.2% | | | | | |
| Refined water | | 0 | 3 | 15 | 2 | Control |

### Example 1

### Hair tonic

Fifty-five parts by weight of ethanol, 2.0 parts of weight of polyoxyethylene (8) oleyl alcohol ether, 35 parts by weight of refined water, 3.0 parts by weight of a pine extract (about 20% concentration), 3.0 parts by weight of a bamboo extract (about 25% concentration), 2.0 parts by weight of a Japanese apricot extract (about 45% concentration), in addition, an adequate amounts of β-thujaplicin (hinokitiol), flavoring agent and coloring agent were mixed in usual manner to obtain a hair restorer in hair tonic form. The present product can be advantageously used in the promotion of the growth and regeneration of hair, as well as in the treatment and prevention of falling out of hair, dandruff, and itching of the scalp or skin.

The product can be also used as a hair tonic because the product exhibits an effect of imparting flavor and refreshment.

In addition, the product can be advantageously used as a preventive or traumatherapeutic agent for the scalp because the product exerts an effect of bactericidal and antiphlogistic activity.

### Example 2

### Hair tonic

Five parts by weight of powdery pine extract, 2.5 parts by weight of bamboo extract (about 40% concentration), 2.0 parts by weight of α-glycosyl rutin ("αG rutin" commercialized by Toyo Sugar Refining Co., Ltd., Japan) and 20.0 parts by weight of glycerin were added to 550 parts by weight of refined water (60°C). The mixture thus obtained was further added a solution containing 0.05 parts by weight of cyanine dyes ("Kankoh-Sikiso No.301") and 440 parts by weight of ethanol, and a solution containing 2.0 parts by weight of L-menthol, 1.0 part by weight of propolis extract (15% concentration) and 10 parts by weight of ethanol, and mixed and filtrated in usual manner and thereafter injected into a bottle to obtain a hair restorer in hair tonic form.

The present product can be advantageously used in the promotion of the growth and regeneration of hair, as well as in the treatment and prevention of falling out of hair, dandruff, and itching of the scalp or skin.

In addition, the product can be advantageously used as a preventive or traumatherapeutic agent for the scalp because the product exerts an effect of bactericidal and antiphlogistic activity.

### Example 3

### Hair liquid

Fifty-five parts by weight of ethanol, 20.0 parts by weight of polyoxyethylene (40) butyl ether, 13 parts by weight of refined water, 5.0 parts by weight of pine extract (about 20% concentration), 5.0 parts by weight of Japanese apricot extract (about 45% concentration) and 3.0 parts by weight of 2-O-α-D-glucopyranosyl-L-ascorbic acid, in addition, an adequate amounts of pH adjuster, flavoring agent and antiseptic, the resultant mixture were mixed to obtain a hair restorer in hair liquid form.

The present product can be advantageously used in the promotion of the growth and regeneration of hair, as well as in the treatment and prevention of falling out of hair, dandruff, and itching of the scalp or skin.

In addition, the present product can be advantageously used as a preventive or traumatherapeutic agent for the scalp because the product exerts an bactericidal and antiphlogistic activity.

### Example 4

### Hair cream

Three parts by weight of beeswax, 15.0 parts by weight of petrolatum, 42.0 parts by weight of liquid paraffin, 3.0 parts by weight of polyoxyethylene (5) ester stearate, 2.0 parts by weight of polyoxyethylene (6) oleyl alcohol ether, 1.0 part by weight of polyoxyethylene (6) cetyl alcohol ether, 1.0 part of weight of powdery pine extract, 1.5 parts by weight of bamboo extract (about 40% concentration), 1.0 part of weight of Japanese apricot extract (about 85% concentration), 0.2 parts by weight of α-glycosyl rutin, 0.3 parts by weight of 2-O-α-D-glucopyranosyl-L-ascorbic acid and 32 parts by weight of refined water, in addition, an adequate of pH adjuster, flavoring agent and antiseptic, were mixed to obtain a hair restorer in hair cream form.

The present product can be advantageously used in the promotion of the growth and regeneration of hair, as well as in the treatment and prevention of falling out of hair, dandruff, and itching of the scalp or skin.

In addition, the product can be advantageously used as a hair cream because the product imparts a gloss to hair, as well as supplementing nutrition to the skin and exerting an antiphlogistic activity.

### Example 5

### Ointment

Ten parts by weight of anionic auto-emulsified wax, 3.0 parts by weight of myristic acid isopropyl, 3.0 parts by weight of liquid paraffin, 4.0 parts by weight of cetanol, 5.0 parts by weight of maltose, 10.0 parts by weight of propolis extract (about 10% concentration), 2.0 parts by weight of a powdery pine extract, 2.0 parts by weight of a bamboo extract (about 20% concentration), 1.0 part by weight of a Japanese apricot extract (about 45% concentration), 1.0 part by weight of α-glycosyl rutin, 51.0 parts by weight of refined water, and an adequate amounts of antiseptic were mixed to obtain a hair restorer in oniment form.

The present product can be advantageously used in the promotion of the growth and regeneration of hair, as well as in the treatment and prevention of falling out of hair, dandruff, and itching of the scalp or skin.

In addition, the present product can be advantageously used as a preventive or traumatherapeutic agent for the scalp because the product exerts an bactericidal and antiphlogistic activity.

### [Effects of the invention]

As described above, the present invention establishes an external-usable hair restorer by incorporating in a pine extract either or both of a bamboo extract and a Japanese apricot extract, and augments extremely an effect of the growth and regeneration of hair which a pine extract exhibits. The present invention has an industrial advantage in the treatment and prevention of alopecia and falling out of hair as well as in the beauty culture and cosmetics. Furthermore, the present invention advantageously contributes for improving the growth and regeneration of hair or plumage of domestic animals and the quality of hair or plumage of pet animals.

## Claims

1. Use of a composition containing as effective ingredients a pine extract and either or both of a bamboo extract and a Japanese apricot extract for the manufacture of a medicament for the restoration of hair loss by the external application of the medicament.

2. A non-therapeutic method of restoring hair of a human which comprises externally applying to said human a composition which contains as effective ingredients a pine extract and either or both of a bamboo extract and a Japanese apricot extract.

3. A use or method according to any preceding claim, wherein the composition contains at least 0.01 w/w% of pine extract and contains 0.1 to 100 fold of bamboo extract and 0.1 to 100 fold of Japanese apricot extract with respect to the quantity of pine extract, respectively, on a dry solid basis.

4. A use or method according to any preceding claim, wherein the composition contains additionally one or more of alpha-glycosyl-L-ascorbic acid, an alpha-glycosyl-bioflavonoid and a propolis extract.

5. A use, or method according to claim 4, wherein said alpha-glycosyl-L-ascorbic acid, is 2-0-alpha-D-glucosyl-L-ascorbic acid, 2-0-alpha-maltosyl-L-ascorbic acid, 2-0-alpha-maltotriosyl-L-ascorbic acid, 2-0-alpha-maltotetraosyl-L-ascorbic acid, 2-0-alpha-maltopentaosyl-L-ascorbic acid, 2-0-alpha-maltohexaosyl-L-ascorbic acid, 2-0-alpha-maltoheptaosyl-L-ascorbic acid, or a mixture thereof.

6. A use, or method according to claim 4 or claim 5, wherein said alpha-glycosyl-bioflavonoid is an alpha-glycosyl rutin, an alpha-glycosyl hesperidin, an alpha-glycosyl naringin, or a mixture thereof.

7. A use or method according to claim 6, wherein said alpha-glycosyl rutin is alpha-glucosyl rutin, alpha-maltosyl rutin, alpha-maltotriosyl rutin, alpha-maltotetraosyl rutin, alpha-maltopentaosyl rutin, or a mixture thereof.

8. A use or method according to claim 6, wherein said alpha-glycosyl hesperidin is alpha-glucosyl hesperidin, alpha-maltosyl hesperidin, alpha-maltotriosyl hesperidin, alpha-maltotetraosyl hesperidin, alpha-maltopentaosyl hesperidin, or a mixture thereof.

9. A use or method according to claim 6, wherein said alpha-glycosyl naringin is alpha-glucosyl naringin, alpha-maltosyl naringin, alpha-maltotriosyl naringin, alpha-maltotetraosyl naringin, or a mixture thereof.

10. A use or method according to any one of claims 5 to 9, wherein the respective amount of said alpha-glycosyl ascorbic acid, alpha-glycosyl bioflavonoid and/or propolis extract is about 0.1 to 100 fold of that of said pine extract, on a dry solid basis.

11. A use or method according to any preceding claim, wherein the composition additionally contains about 0.01 to 100 fold of a cyanine dye with respect to the quantity of said pine extract, d.s.b.

12. A use or method according to any preceding claim, wherein the composition is in the form of liquid, jelly, emulsion, aerosol or ointment.

13. A use or method according to any preceding claim, wherein the composition additionally contains an effective amount of humectant.

14. A use or method according to claim 13, wherein said humectant is propyleneglycol, glycerine or polyoxyethylene glycol.

## Patentansprüche

1. Verwendung einer Zusammensetzung, die als wirksame Bestandteile einen Pinienextrakt, und Bambusextrakt und einen japanischen Aprikosenextrakt, entweder allein oder in Kombination, für die Herstellung eines Medikaments für die Regenerierung von Haarausfall durch äußere Anwendung des Medikaments.

2. Nicht-therapeutisches Verfahren zur Regenerierung des Haars beim Menschen, bei dem eine Zusammensetzung, die als wirksame Bestandteile einen Pinienextrakt, und einen Bambusextrakt und einen japanischen Aprikosenextrakt, entweder einzeln oder in Kombination, enthält, extern bei diesem Menschen angewendet wird.

3. Verwendung oder Verfahren nach einem der vorangegangenen Ansprüche, worin die Zusammensetzung mindestes 0,01 G/G-% Pinienextrakt enthält und das 0,1 bis 100-fache Bambusextrakt und das 0,1 bis 100-fache japanischen Aprikosenextrakt, bezogen auf die Menge des Pinienextrakts auf trockener Feststoffbasis enthält.

4. Verwendung oder Verfahren nach einem der vorangegangenen Ansprüche, worin die Zusammensetzung zusätzlich eine oder mehr Verbindungen aus α-Glycosyl-L-ascorbinsäure, α-Glycosylbioflavonoid und Propolisextrakt enthält.

5. Verwendung oder Verfahren nach Anspruch 4, worin die α-Glycosyl-L-ascorbinsäure 2-0-α-Glucosyl-L-ascorbinsäue, 2-0-α-Maltosyl-L-ascorbinsäure, 2-0-α-Maltotriosyl-L-ascorbinsäure, 2-0-α-Maltotetraosyl-L-ascorbinsäure, 2-0-α-Maltopentaosyl-L-ascorbinsäure, 2-0-α-Maltohexaosyl-L-ascorbinsäure, 2-0-α-Maltoheptaoxyl-L-ascorbinsäure oder eine Mischung daraus ist.

6. Verwendung oder Verfahren nach Anspruch 4 oder Anspruch 5, worin das α-Glycosylbioflavonoid ein α-Glycosylrutin, α-Glycosylhesperidin, α-Glycosylnaringin oder eine Mischung daraus ist.

7. Verwendung oder Verfahren nach Anspruch 6, worin das α-Glycosylrutin ein α-Glycosylrutin, α-Maltosylrutin, α-Maltotriosylrutin, α-Maltotetraosylrutin, α-Maltopentaosylrutin oder eine Mischung daraus ist.

8. Verwendung oder Verfahren nach Anspruch 6, worin das α-Glycosylhesperidin α-Glucosylhesperidin, α-Maltosylhesperidin, α-Maltotriosylhesperidin, α-Maltotetraosylhesperidin, α-Maltopentaosylhesperidin oder eine Mischung daraus ist.

9. Verwendung oder Verfahren nach Anspruch 6, worin das α-Glycosylnaringin α-Glucosylnaringin, α-Maltosylnaringin, α-Maltotriosylnaringin, α-Maltotetraosylnaringin oder eine Mischung daraus ist.

10. Verwendung oder Verfahren nach einem der Ansprüche 5 bis 9, worin die jeweilige Menge α-Glycosylascorbinsäure, α-Glycosylbioflavonoid und/oder Propolisextrakt etwa das 0,1 bis 100-fache derjenigen des Pinienextrakts auf trockener Feststoffbasis ausmacht.

11. Verwendung oder Verfahren nach einem der vorangegangenen Ansprüche, worin die Zusammensetzung zusätzlich etwa das 0,01 bis 100-fache eines Cyaninfarbstoffs, bezogen auf die Menge des Pinienextrakts, d.s.b., enthält.

12. Verwendung oder Verfahren nach einem der vorangegangenen Ansprüche, worin die Zusammensetzung in Form einer Flüssigkeit, eines Gelees, einer Emulsion, eines Aerosols oder einer Salbe vorliegt.

13. Verwendung oder Verfahren nach einem der vorangegangenen Ansprüche, worin die Zusammensetzung zusätzliche eine wirksame Menge eines Feuchtigkeitsmittels enthält.

14. Verwendung oder Verfahren nach Anspruch 13, worin das Feuchtigkeitsmittel Propylenglycol, Glyzerin oder Polyoxyethylenglykol ist.

## Revendications

1. Utilisation d'une composition contenant, comme ingrédients efficaces, un extrait de pin et l'un de, ou les deux, constituants consistant en un extrait de bambou et un extrait d'abricot japonais pour la fabrication d'un médicament pour la régénération capillaire après la chute des cheveux par application externe du médicament.

2. Procédé non thérapeutique de régénération capillaire chez l'Homme, comprenant l'application externe chez ledit homme d'une composition qui contient, comme ingrédients efficaces, un extrait de pin et l'un de, ou les deux, constituants consistant en un extrait de bambou et un extrait d'abricot japonais.

3. Utilisation ou procédé selon l'une quelconque des revendications précédentes, dans laquelle/lequel la composition contient au moins 0,01 % en poids/poids d'extrait de pin et contient de 0,1 à 100 fois la quantité d'extrait de bambou et de 0,1 à 100 fois la quantité d'extrait d'abricot japonais, respectivement par rapport à la quantité d'extrait de pin, sur la base des solides secs.

4. Utilisation ou procédé selon l'une quelconque des revendications précédentes, dans laquelle/lequel la composition contient, en outre, un ou plusieurs acides alpha-glycosyl-L-ascorbiques, un alpha-glycosyl-bioflavonoïde et un extrait de propolis.

5. Utilisation ou procédé selon la revendication 4, dans laquelle/lequel ledit acide alpha-glycosyl-L-ascorbique est l'acide 2-0-alpha-D-glucosyl-L-ascorbique, l'acide 2-0-alpha-maltosyl-L-ascorbique, l'acide 2-0-alpha-maltotriosyl-L-ascorbique, l'acide 2-0-alpha-maltotétraosyl-L-ascorbique, l'acide 2-0-alpha-maltopentaosyl-L-ascorbique, l'acide 2-0-alpha-maltohexaosyl-L-ascorbique, l'acide 2-0-alpha-maltoheptaosyl-L-ascorbique ou un mélange de ceux-ci.

6. Utilisation ou procédé selon la revendication 4 ou la revendication 5, dans laquelle/lequel ledit alpha-glycosyl-bioflavonoïde est une alpha-glycosyl-rutine, une alpha-glycosyl-hespéridine, une alpha-glycosyl-naringine ou un mélange de celles-ci.

7. Utilisation ou procédé selon la revendication 6, dans laquelle/lequel ladite alpha-glycosyl-rutine est l'alpha-glucosyl-rutine, l'alpha-maltosyl-rutine, l'alpha-maltotriosyl-rutine, l'alpha-maltotétraosyl-rutine, l'alpha-maltopentaosyl-rutine ou un mélange de celles-ci.

8. Utilisation ou procédé selon la revendication 6, dans laquelle/lequel ladite alpha-glycosyl-hespéridine est l'alpha-glucosyl-hespéridine, l'alpha-maltosyl-hespéridine, l'alpha-maltotriosyl-hespéridine, l'alpha-maltotétraosylhespéridine, l'alpha-maltopentaosyl-hespéridine ou un mélange de celles-ci.

9. Utilisation ou procédé selon la revendication 6, dans laquelle/lequel ladite alpha-glycosyl-naringine est l'alpha-glucosyl-naringine, l'alpha-maltosyl-naringine, l'alpha-maltotriosyl-naringine, l'alpha-maltotétraosyl-naringine ou un mélange de celles-ci.

10. Utilisation ou procédé selon l'une quelconque des revendications 5 à 9, dans laquelle/lequel la quantité respective desdits acide alpha-glycosyl-ascorbique, alpha-glycosyl-bioflavonoïde et/ou extrait de propolis vaut environ 0,1 à 100 fois la quantité dudit extrait de pin, sur la base des solides secs.

11. Utilisation ou procédé selon l'une quelconque des revendications précédentes, dans laquelle/lequel la composition contient, en outre, un colorant cyanine à raison d'environ 0,01 à 100 fois la quantité dudit extrait de pin, b.s.s.

12. Utilisation ou procédé selon l'une quelconque des revendications précédentes, dans laquelle/lequel la composition se présente sous la forme d'un liquide, d'une gelée, d'une émulsion, d'un aérosol ou d'un onguent.

13. Utilisation ou procédé selon l'une quelconque des revendications précédentes, dans laquelle/lequel la composition contient, en outre, une quantité efficace d'agent hydratant.

14. Utilisation ou procédé selon la revendication 13, dans laquelle/lequel ledit agent hydratant est du propylèneglycol, du glycérol ou du polyoxyéthylèneglycol.
